# EUROPEAN PATENT APPLICATION

(11) **EP 1 180 370 A1**
(43) Date of publication of application: **20.02.2002**
(21) Application number: 00116285.8
(22) Date of filing: 10.08.2000
(51) Int. Cl.: A61L 15/00, A61L 15/22, A61L 15/42, B32B 27/12

(54) **Disposable, moisture vapour permeable, liquid impermeable covering sheet for bedding articles having an improved comfort**

(71) Applicant: The Procter & Gamble Company, Cincinnati, Ohio 45202 (US)
(72) Inventor: Corzani, Italo, 66100 Chieti (IT); Spina, Enrico, 65100 Pescara (IT); Sirianni, Giuseppe, 00165 Rome (IT); Russo, Elisabetta, 00142 Rome (IT); Carrara, Giovanni, 00121 Rome (IT)
(74) Representative: Veronese, Pancrazio

(57) **Abstract**

The present invention relates to a disposable covering sheet for bedding articles which is moisture vapour permeable and liquid impermeable and comprises at least a moisture vapour permeable fibrous layer and a liquid impermeable but moisture vapour permeable film layer of preferred thermoplastic compositions. The disposable covering sheet such as, for example, a mattress cover or a pillow cover provides a better comfort to the user. The disposable covering sheet of the present invention can find a variety of applications wherein moisture vapour permeability and liquid imperviousness are desirable combined with easy disposability and increased in-use comfort.

## Description

### Field of the Invention

The present invention relates to a disposable covering sheet for bedding items such as mattresses, pillows, etc. which is moisture vapour permeable and liquid impermeable and comprises improved liquid impermeable formed structures having an enhanced moisture vapour permeability comprising thermoplastic compositions preferably applied onto a moisture vapour permeable substrate. The covering sheet of the present invention can find a variety of applications wherein moisture vapour permeability, liquid imperviousness, disposability, and comfort are desirable. Preferably the covering sheet of the instant invention is a mattress cover or a pillow cover.

### Background of the Invention

Covering sheets for bedding articles, particularly bed mattress covers, are known in the art. A primary use of these articles is to protect mattresses, particularly bed mattresses, but also pillows, cushions, duvets, upholstery, from contaminants, e.g. from dust, liquids, or bodily fluids, for example in environments where a mattress is intended to be used by different users, such as in hospitals, hotels, or rental houses. Furthermore, mattress covers are used in house for providing a mattress with a protective covering.

More recently mattress covers have been also proposed to act as an allergen barrier to control house mites that live in the dust, typically on and within mattresses where they can find favourable environmental conditions in terms of temperature and nourishment.

It has long been established that house mites, also known as dust mites, are a source of house dust allergens that not only cause allergies, but also adversely contribute to other pathologies, such as asthma. It has also been established that use of allergen control measures is effective in controlling these conditions. Allergen-proof encasing to contain mites to prevent allergen egress has long been used in mattresses, such as bed mattresses, pillows, cushions, duvets, upholstery.

Mattress covers come in direct or indirect (i.e. through further intermediate layers, e.g. bed sheets, or pillowcases) contact with the human body, therefore it is important that such covers are moisture vapour permeable for comfort reasons, in addition to being liquid impermeable in order to provide the mattress with the desired protection against external agents. When used as a barrier against dust mites they should also have an allergen barrier capability.

Various examples of disposable mattress covers which are at the same time liquid impermeable and breathable, i.e. moisture vapour permeable, are known in the art.

Known disposable mattress covers comprise different materials or structures which are capable of providing a liquid barrier, in addition to providing moisture vapour permeability, preferably air permeability. Such structures or materials can comprise a single layer, or multiple layers laminated together. An example are structures comprising thermoplastic microporous films, e.g. laminated to fibrous layers such as nonwoven layers.

Particularly preferred materials which are suitable for disposable mattress covers are hydrophilic continuous films, also known as "monolithic films", that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower.

In our patent applications EP 0963760 and EP 0964026 thermoplastic compositions are disclosed for making hydrophilic continuous moisture vapour permeable, liquid impermeable layers having preferred characteristics of moisture vapour permeability and liquid imperviousness. The disclosed preferred thermoplastic compositions are also readily processable so as to provide a coating having the desired thickness onto a substrate, so avoiding the need of complex traditional extrusion apparatuses. This is achieved by modifying the viscosity of the thermoplastic polymers by means of the inclusion in the composition of a suitable plasticiser or blend of plasticisers that lowers such viscosity. This allows to utilise with these preferred compositions typical process conditions known in the art for the direct coating of low viscosity hot melt compositions onto a substrate in order to form a moisture vapour permeable, liquid impervious film or layer.

Disposable, moisture vapour permeable, liquid impermeable mattress covers comprising a moisture vapour permeable, liquid impermeable structure, which in turn comprises a layer of the thermoplastic compositions according to either EP 0963760 or EP 0964026, preferably applied onto a moisture vapour permeable substrate, e.g. a fibrous layer such as a nonwoven layer in order to form a composite structure, are particularly preferred since they provide better moisture vapour permeability combined with liquid imperviousness, and also take advantage of the increased ease of manufacture associated to the above mentioned compositions.

Another improvement to disposable mattress covers is described in our European patent application No. 99124535.8, filed on 9 December 1999, where an increased friction is established between the disposable mattress cover and the mattress surface during the use, when i.e. the mattress cover is typically subjected to a certain compression against the mattress itself, in order to minimise possible misplacements of the cover during its use, for example when the mattress is a bed mattress or a pillow, and the mattress cover can be moved or misplaced by the user's movement, e.g. during the sleep. This is particularly important when the mattress cover is intended for partial covering of the external surface of a mattress, for example of the upper surface of a bed mattress, e.g., in combination with traditional means for connecting the cover to the mattress, such as for example elastic bands or strands applied at the corners of a rectangular bed mattress cover.

Provision of an increased friction between the mattress cover and the mattress is however also useful in the context of a mattress cover intended for total encasing of a mattress. In both cases in fact this increased friction prevents or at least reduces relative movements between the mattress cover and the mattress during the use, therefore helping in keeping the mattress cover in its right position provided initially, e.g. by the user when applying the cover to the mattress.

Even if the bedding items disclosed in our above mentioned patent applications function well, particularly in terms of protection, it was surprisingly discovered by selecting some parameters of the components constituting the bedding articles of the present invention as thereafter indicated, and preferably by using certain thermoplastic compositions for the hydrophilic continuous layer comprised in the bedding article, as described in the European patent application having the title "Thermoplastic hydrophilic polymeric compositions with improved adhesive properties for moisture vapour permeable structures" (P&G Case CM2406F, filed on the same day of the present invention) the comfort for the user is significantly increased compared to the prior art items, while offering the same or better protection.

### Summary of the Invention

A disposable, moisture vapour permeable, liquid impermeable, covering sheet for bedding articles comprising at least a moisture vapour permeable fibrous layer and a liquid impermeable but moisture vapour permeable film layer applied onto the fibrous layer, the covering sheet having the following characteristics:
- a Water Vapour Transmission Rate (WVTR) value of at least 400 g/m²• 24h;
- a Hydrostatic water Head (HH) value of at least 1 cm;
- a ratio R of the caliper, expressed in micrometers, to the basis weight, expressed in g/m², of at least 2.5.

The Water Vapour Transmission Rate, the Hydrostatic water Head, and the caliper are measured according to the methods described herein.

### Brief Description of the Drawings

The drawings herein are intended to illustrate preferred embodiments of the present invention, but are not meant to limit in any way its scope.
Figure 1 is a schematic plan view of a disposable mattress cover according to the present invention.
Figure 2 is a cross-sectional view of the disposable mattress cover of figure 1 taken along the line A-A.
Figure 3 illustrates the mattress cover applied to a mattress.

### Detailed Description of the Invention

According to the present invention, a disposable, moisture vapour permeable, liquid impermeable covering sheet for bedding articles is provided which comprises at least a moisture vapour permeable fibrous layer and a liquid impermeable but moisture vapour permeable film layer comprising a highly processable thermoplastic composition having an enhanced moisture vapour permeability. Said covering sheet offers an increased comfort to the user, particularly when used as a mattress cover.

The term "mattress", as used therein, refers to a fabric case filled with resilient material, such as for example cotton, hair, feathers, foam rubber, or an arrangement of coil springs, and therefore comprises particularly bed mattresses, but also pillows, cushions, comforters, duvets, upholstered portions of beds (such as headboards), or of sofas or armchairs. Consequently, even if the present invention will be described with particular reference to a disposable covering sheet for bedding items in the form of a mattress cover, it is not meant to limit in any way its scope to mattress covers.

Referring particularly to mattresses it is generally known that each mattress every year "absorbs" from about 45 kilos to about 80 kilos of sweat and about 150 grams of skin scales. In addition to this, millions of mites generally live in the mattress. Another problem is that accidental staining is practically unavoidable. Usually consumers are aware of the above problems and, in fact, most of them use reusable mattress covers which usually are made from cotton. But these covers do not solve the above problems.

An ideal mattress cover should offer a number of benefits such as a superior protection, i.e. by constituting a barrier to stains, dust, allergens, and micro organisms; a superior in-use comfort, i.e. by being breathable or water vapour permeable (no sweat), by producing a low noise in use, and by providing a softness sensation to the user; and a superior ease of use, i.e. it by being disposable so as to avoid reuse and high temperature laundering.

It should be noted that disposability involves not only a light and low cost structure but also an easy application, removal and disposal.

Contrary to the prior art the present invention solves the above problems by providing a disposable covering sheet for bedding articles and particularly a disposable mattress cover with superior protection, superior in-use comfort, and ease of use.

The disposable covering sheet for bedding articles of the present invention consists in a laminate of different layers comprising at least a first moisture vapour permeable layer and a second liquid impermeable but moisture vapour permeable film layer of thermoplastic composition bonded onto said first layer, i.e. the laminate consists of at least two bonded layers giving rise to a composite structure.

If more film layers of thermoplastic compositions are utilised in a composite structure in combination with one or more other moisture vapour permeable layers to create a layered moisture vapour permeable, liquid impermeable composite structure, each film layer can be made of a same specific thermoplastic composition according to the present invention, or of different specific thermoplastic compositions according to the present invention.

The above composite structure can involve at least one component, typically the film layer, of the thermoplastic composition in combination with one or more other moisture vapour permeable layers. Such moisture vapour permeable materials include, but are not limited to: fibres, fibrous batts, non-wovens, wovens, papers, micro-porous or porous membranes, films such as polymeric films, perforated or apertured films and papers, macroscopically expanded films, cloth, etc.

Said other components may be non-absorbent, absorbent, liquid-containing, etc.

The composite structures of the covering sheets described above have a moisture vapour transfer rate (WVTR) of at least 400 g/m²•24h, more preferably at least 500 g/m²•24h, and most preferably at least 600 g/m²•24h. They have a Hydrostatic water Head (HH) value of at least 1 cm, preferably of at least 10 cm, and a ratio (R) of caliper, expressed in micrometers, to the basis weight (expressed in g/m²) of at least 2.5, preferably of at least 3.5.

According to a particularly preferred embodiment of the present invention the disposable, moisture vapour permeable, liquid impermeable mattress cover comprises a composite structure comprising a film layer of the thermoplastic composition applied onto a moisture vapour permeable substrate, wherein said film layer is intended, in use, to directly contact the mattress. The moisture vapour permeable substrate preferably comprises a fibrous layer, e.g. a nonwoven layer onto which the thermoplastic composition is applied in a continuous layer or film by means of known means, e.g. preferably by hot melt coating.

A disposable, moisture vapour permeable, liquid impermeable mattress cover comprising a moisture vapour permeable, liquid impermeable structure in turn comprising a layer of the thermoplastic composition according to the present invention which is intended, in use, to directly contact a mattress, can have any shape and arrangement which is known in the art, and which is suitable for its intended use.

For example, if the mattress consists of a standard bed mattress, the mattress cover can be a substantially rectangular flat sheet with elastic strings at the corners, or alternatively with integrally elasticised corners, as described in FR 2747899. The rectangular sheet typically has suitable dimensions in order to completely cover at least the upper surface of the bed mattress, and preferably, as in the latter embodiment referred to above, also the side surfaces thereof, wherein the elastic strings or the elasticised corners provide for a suitable releasable connection, in a way known in the art, to the mattress. Alternative configurations where the mattress cover has a suitable shape and size in order to completely encase a mattress are also possible. This latter configuration could be preferred when the mattress cover, in addition to liquid imperviousness and moisture vapour permeability, should also to provide a total barrier against dust mites and related allergens.

Preferably, as shown in the herein drawings, a disposable mattress cover comprises a rectangular blanket made according to the present invention. In particular, the disposable, moisture vapour permeable, liquid impermeable blanket is formed by a topsheet 1, which is a moisture vapour permeable fibrous layer, and a backsheet 2, which is a liquid impermeable but moisture vapour permeable film layer of a thermoplastic composition, according to the present invention, laminated onto said topsheet 1 in order to form a two-layer composite structure. The backsheet 2 is intended to face and to be in direct contact with the top of a mattress 4, while the topsheet 1 is intended to face the user.

The thermoplastic compositions of the present invention can provide a certain degree of residual tackiness to the backsheet 2. In turn, this tackiness prevents or at least reduces relative movements between the mattress cover and the mattress 4 during use, therefore helping in keeping the mattress cover in its right position provided by the user initially. In any case and if preferred, the mattress cover can have no residual tackiness by suitably selecting the thermoplastic compositions according to the present invention or by neutralising any residual tackiness with known means, e.g. talcum powder.

For assisting the user to put the mattress cover on the mattress in the right position but also for fixing the mattress cover to the mattress in a stable manner four adhesive means 3 are provided, as shown in the drawings. The adhesive means 3 can be one or more stripes of a conventional pressure sensitive adhesive covered by a release liner for protecting the adhesive before use, as utilised, for example, in the manufacture of the panty fastening adhesive patch of commercial sanitary napkins.

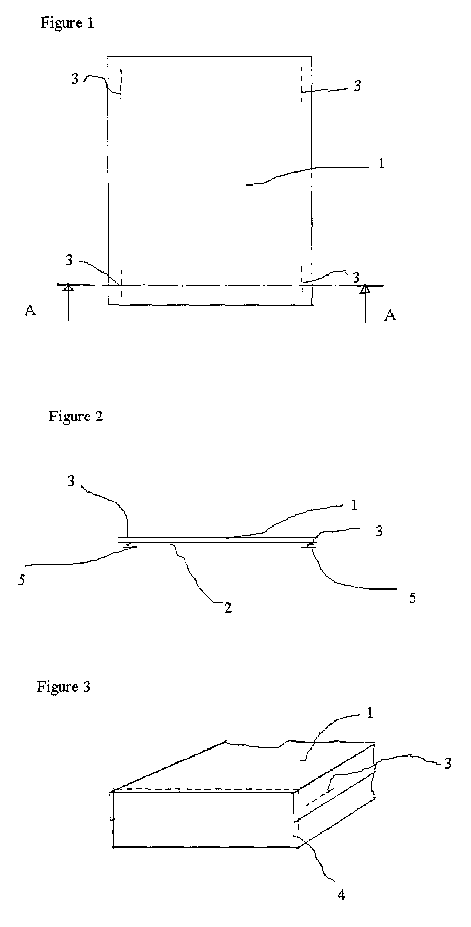

As shown in figure 3, the mattress cover has suitable dimensions to substantially or completely cover at least the top surface of the mattress 4, while the adhesive means 3 are preferably attached to the lateral sides of the mattress 4.

Any other suitable shape or arrangement for a mattress cover according to the present invention is also possible, depending on the particular mattress type, shape and dimensions, and on the intended use of the mattress cover.

Preferably the disposable, moisture vapour permeable, liquid impermeable covering sheet for bedding articles of the present invention, when used as a mattress cover or a pillow cover, has a surface area ranging from 15.0 m² to 0.3 m², preferably from 8.0 m² to 0.5 m². Most preferably, particularly in the case of a mattress cover, said surface area is from 4.0 m² to 1.0 m².

### The thermoplastic compositions

According to a preferred embodiment of the present invention, the thermoplastic polymeric composition for making a moisture vapour permeable, liquid impervious structure at least comprises a thermoplastic hydrophilic polymer or a mixture of thermoplastic hydrophilic polymers, and a suitable compatible hydrophilic tackifying resin, or a blend of suitable compatible hydrophilic tackifying resins, in order to provide a thermoplastic polymeric hydrophilic composition with a desired degree of adhesiveness or tackiness in the molten, semi-molten, or plastic state, and/or in the stable state at room temperature.

In the following description the terms "adhesiveness" and "tackiness" are considered to be synonymous.

The terms "breathable" and "breathability" are intended herein to correspond to "moisture vapour permeable" or "water vapour permeable", and "moisture vapour permeability" or "water vapour permeability". "Moisture vapour" and "water vapour" are also considered to be equivalent.

Suitable thermoplastic hydrophilic polymers comprised in the composition according to the present invention include polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, poly lactide and copolymers, polyamides, polyesters and copolyesters, polyester block copolymers, sulfonated polyesters, poly-ether-ester block copolymers, poly-ether-ester-amide block copolymers, polyacrylates, polyacrylic acids and derivatives, ionomers,_polyethylene-vinyl acetate with a vinyl acetate content of more than 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, and mixtures thereof.

Particularly preferred thermoplastic hydrophilic polymers are thermoplastic poly-ether-amide block copolymers (e.g. Pebax™), thermoplastic poly-ether-ester-amide block copolymers, thermoplastic polyester block copolymers (e.g. Hytrel™), thermoplastic polyurethanes, typically non reactive polyurethanes (e.g. Estane™), or mixtures thereof.

The hydrophilic tackifying resin comprised in the thermoplastic hydrophilic polymeric compositions according to this preferred embodiment of the present invention must have a high polar character, wherein said polar character is measured and expressed by means of a suitable polarity parameter.

When alternatively a blend of hydrophilic tackifying resins is comprised in the thermoplastic hydrophilic polymeric compositions of the present invention, said blend must comprise at least 50% by weight of a tackifying resin or resins having said high polar character. Preferably each hydrophilic tackifying resin of the blend has said high polar character.

A suitable polarity parameter can be selected among parameters known in literature which are useful to give a measure of the polar character of a substance, for example in terms of its hydrophilicity or of its solubility, typically in water, measured by means of suitable test methods. For example, known polarity parameters suitable for measuring the polar character of a substance in the context of the present invention comprise the water absorption at equilibrium, or the Hildebrand solubility parameter, or the contact angle with water, or the dual contact angle, which parameters are well known in physical chemistry and are measurable according to known methods which can be found in the scientific and technical literature.

It has been discovered that a particularly suitable polarity parameter for measuring the polar character of a tackifying resin according to this preferred embodiment of the present invention is the equilibrium contact angle with water of the hydrophilic tackifying resin, measured according to the Equilibrium Contact Angle Measuring Test method described herein. The equilibrium contact angle with water actually gives a measure of the hydrophilicity of a substance in terms of the interaction between that substance and a drop of water at a common interface.

According to the present invention, the hydrophilic tackifying resin has the required high polar character according to the present invention if it has an equilibrium contact angle with water not greater than 86 degrees, preferably not greater than 84 degrees, more preferably not greater than 82 degrees, as measured according to the Equilibrium Contact Angle Measuring Test described herein.

Alternatively, if a blend of compatible hydrophilic tackifying resins is comprised in the thermoplastic hydrophilic polymeric composition of the present invention, said blend must comprise at least 50% by weight of a tackifying resin or resins having an equilibrium contact angle with water not greater than 86 degrees, preferably not greater than 84 degrees, more preferably not greater than 82 degrees, as measured according to the Equilibrium Contact Angle Measuring Test described herein.

Preferably however, each compatible hydrophilic tackifying resin of such a blend should have the required high polar character expressed in terms of its preferred equilibrium contact angle with water as explained above.

Suitable compatible hydrophilic tackifying resins, or blends of hydrophilic tackifying resins, having the required high polar character compatible with the thermoplastic hydrophilic polymer or mixture of thermoplastic hydrophilic polymers according to the present invention can be selected by the man skilled in the art among available tackifying resins by measuring the value of the respective polarity parameter, namely the equilibrium contact angle with water as explained above, and according to_the test method described herein. Suitable resins are for example the tackifying resins produced by Hercules Inc. and available under the codes Res A-2690, Res A-2691, Res A-2682, Res A-2683, Res A-2698.

The selected preferred hydrophilic tackifying resin or blend of hydrophilic tackifying resins having high polar character comprised in the thermoplastic hydrophilic polymeric compositions of the present invention are generally such that, when such a hydrophilic tackifying resin is added at a level of 30% by weight to a pure thermoplastic hydrophilic polymer to form a two component thermoplastic hydrophilic polymeric composition, the water vapour transmission rate (WVTR) of a 50 µm thick continuous film formed from the thermoplastic hydrophilic polymeric composition constituted by the hydrophilic tackifying resin and the hydrophilic polymer is_at least 40%, preferably at least 50%, more preferably at least 60% of the WVTR value of a 50 µm thick continuos film made of the pure hydrophilic polymer and not comprising the tackifying resin, wherein the WVTR values are measured according to the modified ASTM E-96 "Upright Cup" Method. 30% by weight corresponds to a typical average addition level for a tackifying resin in the thermoplastic hydrophilic polymeric compositions of the present invention.

Preferably the thermoplastic hydrophilic polymeric compositions according to the present invention comprise a blend of hydrophilic tackifying resins, more preferably with each resin of the blend being selected by means of the polarity parameter satisfying the conditions as described above, wherein the blend comprises at least a hydrophilic tackifying resin which is liquid at room temperature, since this is beneficial to the processability of the thermoplastic hydrophilic polymeric composition._Preferably not all the tackifying resins of such a blend are however liquid at room temperature.

By saying "a tackifying resin liquid at room temperature" it is herein meant a tackifying resin having a softening point below 25°C, wherein said softening point is evaluated according to the Ring and Ball Method ASTM E 28-67. Conversely a tackifying resin which is solid at room temperature is a resin having a softening point above 25°C. A temperature of 25°C has been considered in this context to conventionally correspond to the "room temperature".

The thermoplastic hydrophilic polymers or mixture of thermoplastic hydrophilic polymers as mentioned above, comprised in the thermoplastic hydrophilic polymeric composition of the present invention, can be typically highly viscous in the molten state at the process conditions that are typical of the known processes of film or layer formation, e.g. an extrusion process involving a high power screw extruder. For example they may have a viscosity higher than 5000 poise at a temperature of 20°C above the DSC (Differential Scanning Calorimetry) melting point, which is the temperature_identified as that corresponding to the DSC peak, or corresponding to the highest DSC peak in case of a mixture of polymers showing more than one peak, and at a frequency of 1 rad/sec.

The thermoplastic hydrophilic polymeric compositions of the present invention, comprising the thermoplastic hydrophilic polymer(s) and the compatible hydrophilic tackifying resin(s), can therefore still be highly_viscous in the molten state at the process conditions.

According to a preferred embodiment of the present invention, and as disclosed in our patent applications EP 0963760 or EP 0964026, the viscosity of the thermoplastic polymeric hydrophilic compositions of the_present invention can be preferably adjusted by including in the thermoplastic hydrophilic polymeric composition a suitable plasticiser, or blend of plasticisers, that is also compatible with the thermoplastic hydrophilic polymer or polymers and with the hydrophilic tackifying resin or resins and that lowers the viscosity of the thermoplastic hydrophilic polymeric composition in the molten state at the process conditions.

Viscosity of the thermoplastic hydrophilic polymeric compositions of the present invention can therefore be adjusted by suitably selecting the plasticiser, depending on how the composition is to be processed. For example film extrusion techniques can be suitably used with compositions having higher viscosity at the process conditions, as it is known in the art. Alternatively, suitable hot melt coating processes can be preferred to process the compositions, as explained in the above mentioned patent applications EP 0963760 and EP 0964026. This implies that the viscosity in the thermoplastic hydrophilic polymeric composition at the process conditions has to be adjusted at a suitable lower level.

In such a case, the thermoplastic polymeric hydrophilic compositions of this alternative embodiment of the present invention comprise a suitable plasticiser or blend of plasticisers such that they preferably have the following complex viscosities (η∗ ):

50 poise < η∗ < 4000 poise, preferably 100 poise < η∗ < 2000 poise, more preferably 100 poise < η∗ < 1000 poise, at a frequency of 1 rad/s at a temperature of 210°C or less and η∗ < 2000 pose, preferably η∗ < 1000 poise, more preferably η∗ < 500 poise, at a frequency of 1000 rad/s at a process temperature (T) of 210°C or less, wherein η∗ represents the complex viscosity of the thermoplastic polymeric hydrophilic composition. Preferably the temperature T is 200°C or less and more preferably 180°C or less and most preferably from 200°C to 50°C.

According to this preferred embodiment of the present invention the thermoplastic hydrophilic polymeric compositions having the complex viscosity described above allow for a film or layer to be coated onto a substrate using typical coating conditions and apparatuses known in the art for the coating of low viscosities hot melt compositions in a layer having a required thickness onto a substrate, while also keeping the advantageous characteristics of the preferred thermoplastic hydrophilic polymers in providing hydrophilic continuous moisture vapour permeable, liquid impermeable layers or films.

Thermoplastic hydrophilic polymeric compositions having such viscosities can also provide very thin films or layers.

Suitable compatible plasticisers comprised in the thermoplastic hydrophilic polymeric composition according to this preferred embodiment of the present invention include citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and_polyglycols and their derivatives, sorbitan esters, phosphates, monocarboxylic fatty acids (C₈-C₂₂) and their derivatives, and mixtures thereof.

According to a particularly preferred embodiment of the present invention particularly preferred plasticisers are hydrophilic plasticisers such as acids, esters, amides, alcohols, polyalcohols, or mixtures thereof, as disclosed in our application WO 99/64505. Said particularly preferred hydrophilic plasticisers have a particularly high polar character and provide the further advantage that they do not impair, and possibly can even enhance, the moisture vapour permeability of the resulting layer or film formed from the preferred thermoplastic hydrophilic polymeric composition of the present invention comprising said plasticiser or blend of plasticisers, when compared to a corresponding film or layer formed from a thermoplastic hydrophilic polymeric composition comprising the same components, but without the plasticiser or plasticisers.

The particularly preferred hydrophilic plasticiser or blend of hydrophilic plasticisers can of course also adjust the viscosity of the thermoplastic composition according to a preferred embodiment of the present invention to the preferred values in order to make it processable by coating said thermoplastic composition onto a substrate in a film layer having a desired thickness.

Suitable preferred hydrophilic plasticisers according to this preferred embodiment of the present invention comprise acids, esters, amides, alcohols, polyalcohols, or mixtures thereof, wherein particularly preferred hydrophilic plasticisers are citric acid esters, tartaric acid esters, glycerol and its esters, sorbitol, glycolates, and mixtures thereof.

A suitable polarity parameter, such as one of those described above, can also be used in order to select the other components of the thermoplastic polymeric hydrophilic compositions of the present invention. Particularly, preferred thermoplastic hydrophilic polymers and, optionally, preferred compatible hydrophilic plasticisers can be selected for the thermoplastic polymeric hydrophilic compositions of the present invention by means of a suitable polarity parameter according to the same principles already explained with reference to the compatible hydrophilic tackifying resins. A same polarity parameter can be used to select different components of the thermoplastic hydrophilic polymeric compositions of the present invention, or alternatively different polarity parameters for different components, as can be determined by the man skilled in the art.

Preferably the thermoplastic polymeric hydrophilic composition of the present invention comprises from 10% to 99%, preferably from 20% to 70%, more preferably from 30% to 50%, by weight of the thermoplastic polymeric hydrophilic composition, of the thermoplastic hydrophilic polymer or mixture of thermoplastic hydrophilic polymers, and from 1% to 90%, preferably from 10% to 70%, more preferably from 20% to 50%, by weight of the thermoplastic hydrophilic composition, of the suitable compatible hydrophilic tackifying resin or blend of hydrophilic tackifying resins.

More preferably the thermoplastic polymeric hydrophilic composition of the present invention also comprises from 0% to 95%, preferably from 10% to 70%, more preferably from 20% to 50% by weight of the thermoplastic polymeric hydrophilic composition, of a suitable compatible plasticiser or blend of suitable compatible plasticisers.

The thermoplastic hydrophilic polymeric compositions of the present invention may in addition comprise additional optional components to further improve the processibility of the compositions and also the mechanical characteristics as well as other characteristics as resistance to ageing by light and oxygen, visual appearance etc., of the films or layers formed from such thermoplastic hydrophilic polymeric compositions.

A thermoplastic hydrophilic polymeric composition according to the present invention can be manufactured with any known process that will typically comprise the steps of providing at least the thermoplastic hydrophilic polymer or mixture of polymers and the suitable compatible hydrophilic tackifying resin or blend of compatible hydrophilic tackifying resins, and optionally any further additional components as explained above, such as for example a plasticiser or blend of plasticisers, heating the components and compounding them, e.g. with a known suitable mixer to form the thermoplastic hydrophilic polymeric composition in the molten state for subsequent process steps.

According to the present invention a moisture vapour permeable, liquid impervious layer can be formed from the thermoplastic hydrophilic polymeric composition of the present invention, for example by laying said thermoplastic hydrophilic polymeric composition onto a substrate. The films or layers formed from the thermoplastic compositions of the present invention preferably have a moisture vapour transmission rate of at least 400 g/m²·24h, more preferably of at least 500 g/m²·24h, even more preferably of at least 600 g/m²·24h, most preferably of at least 1000 g/m²·24h, with a thickness of said layer or film of at least 40 µm, said water vapour transmission rate measured according to the modified ASTM E-96 "Upright Cup" Method. More preferably, the films or layers as described above have the above preferred WVTR levels wherein the thermoplastic hydrophilic polymeric composition comprises at least 30% by weight of the hydrophilic tackifying resin, or of a blend of hydrophilic tackifying resins, according to the present invention.

### Other characteristics

According to the present invention a moisture vapour permeable, liquid impervious layer can be formed from the thermoplastic composition of the present invention by coating said thermoplastic composition onto a substrate.

A process for making a film layer from a thermoplastic composition according to the present invention typically comprises the steps of providing said composition, heating it to make it flowable, and coating said composition in the molten state onto a substrate in a layer having the desired thickness. In accordance with the present invention, a moisture vapour permeable, water impervious composite is formed which comprises the thermoplastic composition and a substrate onto which said thermoplastic composition is coated, wherein the substrate is also moisture vapour permeable.

Such embodiment provides a moisture vapour permeable, liquid impervious composite structure wherein the film layer formed from the thermoplastic composition of the present invention is firmly bonded to the substrate while providing the required performance for liquid barrier and hence could be advantageously provided as thinly as possible. In this manner such embodiment provides a moisture vapour permeable, liquid impervious composite structure wherein the contribution of the film layer formed from the thermoplastic composition of the present invention to the performance of the composite material resides mainly in the provision of a liquid barrier. The remaining performance physical criterion being preferably provided by the provided substrate, that therefore preferably acts also as a support layer. However it should be noted that the film layer does not need to be as thin as possible in any application because the thermoplastic compositions of the present invention provide high breathability (high WVTR) also in the case of thicker film layers. Typical thickness of the film layer of the thermoplastic composition applied onto a suitable substrate to form a composite structure according to the present invention range from 2 µm to 200 µm.

It should be appreciated that the above modalities of forming the composite structure in combination with the composition of the film layer according to the present invention, allow to provide a disposable covering sheet for bedding articles offering a superior protection, a superior in-use comfort, and an ease of use.

In particular, the preferred adhesiveness or tackiness imparted to the thermoplastic hydrophilic polymeric composition of the present invention by the addition of the compatible hydrophilic tackifying resin or blend of hydrophilic tackifying resins in fact typically provides for an increased adhesion of the film or layer in the molten, semi-molten, or plastic state to the substrate, for example a fibrous substrate such as a nonwoven layer comprising hydrophobic synthetic fibres, while at the same time keeping a high breathability of the film or layer, and hence preferably of the whole layered structure. This in turn provides a better integrity of the resulting composite structure, which is therefore more resistant to e.g. delamination in use, also with very thin layers of the thermoplastic hydrophilic polymeric composition, wherein said improved adhesive properties of the composition and said better resistance of the resulting composite structure are combined with a very limited, or no detrimental effect at all on the water vapour transmission capability of the layer formed from the thermoplastic hydrophilic polymeric composition of the present invention, for example if compared to a layer of the same thickness formed from a similar composition, not comprising the hydrophilic tackifying resin or blend of hydrophilic tackifying resins of the present invention.

Furthermore preferred hydrophilic tackifying resins according to the present invention can also increase the water vapour transmission rate of a layer formed from the thermoplastic hydrophilic polymeric composition of the present invention, when compared to a layer of the same thickness formed from a similar composition, not comprising the hydrophilic tackifying resin or blend of hydrophilic tackifying resins of the present invention.

The thermoplastic hydrophilic polymeric compositions for making moisture vapour permeable, liquid impermeable structures according to the present invention have been so far described as being provided with the desired adhesiveness or tackiness typically in the molten, semi-molten, or plastic state. This is desired in a preferred embodiment of the present invention in order to form e.g. more stable moisture vapour permeable, liquid impermeable layered composite structures with the thermoplastic hydrophilic polymeric composition directly formed as a layer or film onto a suitable substrate, for example substrates having a very low polar character such as preferred nonwovens comprising hydrophobic fibres, wherein said increased adhesiveness to a substrate is not achieved to the detriment of the moisture vapour permeability of the resulting layer or film.

However, the thermoplastic hydrophilic polymeric compositions of the present invention can also be formulated in order to have pressure sensitive adhesive character, i.e. such that the thermoplastic hydrophilic polymeric composition remains tacky at any temperature.

The substrate, or support layer may be any useful layer which is also moisture vapour permeable, preferably having a moisture vapour permeability of at least 400 g/m²•24h, more preferably at least 500 g/m²•24h, and most preferably at least 600 g/m²•24h.

Preferred support layers for use herein include woven and nonwoven layers, most preferably hydrophobic fibrous layers such as hydrophobic nonwoven. As for non limiting examples, a SMS (Spunbonded-Meltblown-Spunbonded) hydrophobic 100% polypropylene nonwoven of 25 g/m² commercialised under the code MD3000 by Corovin - BBA Nonwovens Group (Germany), or a spunlaced nonwoven of 50 g/m² commercialised under the code TJET C500 by Tecnofibra (Italy) function well.

Suitable substrates for use herein as further layers in the structure according to the present invention could also include two dimensional, planar micro and macro-porous films; macroscopically expanded films; formed apertured films; nonwoven and woven layers. Suitable macroscopically expanded films for use herein include films as described in for example in US 4,637,819 and US 4,591,523.

The structures of the preferred embodiment according to the present invention are particularly advantageous as they allow the possibility of providing a composite wherein the thermoplastic composition may be applied onto the support substrate as a film layer with the desired thickness. Typical coating conditions and apparatuses known in the art for the direct coating of low viscosities hot melts can be readily utilised in order to provide the thermoplastic composition at the desired thickness.

A possible method for forming a composite laminate by coating the thermoplastic composition onto a substrate acting as a support layer is described in PCT application WO 96/25902.

At least at the coating temperature, the thermoplastic composition in form of a layer preferably exhibits adhesive properties on the supportive substrate in order to form the preferred composite such that no additional adhesive is required to achieve a permanent attachment between the thermoplastic composition and the substrate.

A moisture vapour permeable, liquid impervious composite structure formed by coating the thermoplastic hydrophilic composition according to the preferred embodiment of the present invention onto a suitable substrate finds particular utility as a structure constituting a disposable, moisture vapour permeable, liquid impermeable mattress cover according to the present invention.

### Example

A polyether-amide block copolymer available from Atofina (France) commercialised under the trade name Pebax 2533 SN is compounded with Tri Butyl Citrate (plasticiser) available from Aldrich Co., Res A-2691 (tackifier resin) available from Hercules Inc., Res A-2690 (tackifier resin) available from Hercules Inc. and Irganox 1010 (anti oxidant agent) available from Ciba-Geigy.

The final formulation in percent by weight has the following composition:

| | |
|---|---|
| 44% | Pebax 2533 SN |
| 25% | Tri Butyl Citrate |
| 15% | Res A-2691 |
| 15% | Res A-2690 |
| 1% | Irganox 1010 |

The blend is melt extruded to obtain a film having a basis weight of 20 g/m². The film is laminated directly onto a substrate constituted by a SMS (Spunbonded-Meltblown-Spunbonded structure) hydrophobic 100% polypropylene nonwoven 25 g/m² (support layer) commercialised under the trade name MD3000, available form Corovin - BBA Nonwovens Group (Germany). The composite has a thickness 230 µm , a ratio R equal to 5.1, a WVTR of 846 g/m²•24h, and a HH value of 17cm.

The composite structure is used to produce a mattress cover, e.g. a bed mattress cover as illustrated in the herein drawings. It has a flat rectangular shape with suitable dimensions to completely cover at least the upper surface of a bed mattress, wherein the film layer 2 of thermoplastic composition is intended to face, in use, the mattress 4, and to directly contact it. In particular, the dimension are: 1.64 m (width) and 1.96 m (length) to completely or substantially completely cover the upper surface of a bed mattress and partially its sides as indicated in figure 3. The four corners of the mattress cover are fixed to the corners of the bed mattress by four adhesive stripes 3, after detaching the release liners 5 which protect the adhesive before use. The adhesive is a conventional pressure sensitive one as normally used, for example, in manufacturing the panty fastening adhesive of sanitary napkins.

### Comparison results

The above disposable mattress cover was compared in internal tests to Intervent ® which is a full encasing reusable mattress cover produced by W. L. Gore & Associati S.r.L. (Italy) having a total surface area of 8.6 m² for a double bed mattress. Its thickness is 250 µm, its basis weight is 130 g/m², and its ratio R is 1.92, its WVTR is 2345 g/m²•24h, and its HH is more than 300 cm.

Two internal tests, one for the evaluation of softness and another for the evaluation of noise were conducted. Each test was a direct comparison between Intervent ® and the mattress cover of the present invention. The test was conducted in the following manner:
- 20 persons (P&G employees) were asked to make a sensorial and qualitative direct comparison of an A4 dimension ( 210 x 297 mm) sheet of Intervent ® (cut from it) and a same dimension sheet of the mattress cover of the present invention as illustrated in the example;
- each person was asked to touch and handle the sheets (samples), first one per time and then together, while the sheets were hidden in two boxes so that the person was unable to see them but only to touch them for avoiding any influence due to any different visual appearance of the two samples.

The sheets were changed with new ones after 3 people had tested them;
- at the end of each test the person gave his preference for the sample he preferred or expressed no preference for both of them.

There were performed two separate tests each involving 20 people, one was for noise and another for softness.

The following results were obtained:

| | Absence of noise (Preference) | Softness (Preference) |
|---|---|---|
| Product according to the present invention | 14 | 11 |
| Intervent ® | 4 | 6 |
| No preference | 2 | 3 |

It is evident from the above data the product according to the present invention was preferred for a better absence of noise and for a better softness which, in turn, combined with the other characteristics of the product according to the invention, give rise to a better in-use comfort.

### Tests methods

According to the present invention the complex viscosity η∗ is_measured using a Rheometer RDA-II available from Rheometrics Co._Moisture vapour permeability is measured as Water Vapour Transmission Rate (WVTR) at 25°C and 55% relative humidity according to the modified ASTM E-96 "Upright Cup" method. The only modification to the standard ASTM E-96 "Upright Cup" method consists in a change in the height of the air gap between the sample and the water surface in the cup, which height is 3 mm ± 0.5 mm, instead of 19 mm ± 2.5 mm, as specified in the standard test method.

The softening point of the resins is measured according to the Ring and_Ball Method ASTM E28 - 67. Thickness or caliper is measured according to the SCAN P-7:55 method. Hydrostatic water head value (HH) is measured according to the DIN 53886 method and reported in cm (centimetres).

### Equilibrium Contact Angle Measuring Test.

The test method, which will be described hereinafter, is intended to measure the equilibrium contact angle with water of a tackifying resin intended to be comprised in a thermoplastic hydrophilic polymeric composition according to the present invention. As it is known in the art, the contact angle is a measure of the phenomenon of wetting or non-wetting of a solid by a liquid. The equilibrium contact angle is measured on a drop of liquid resting in equilibrium on the surface of a substance, wherein the surface is horizontal. The angle between the baseline of the drop and the tangent at the drop boundary is measured.

### Apparatus

Climatically controlled laboratory maintained at 25°C and 55% relative humidity.

Drop Shape Analysis System DSA 10 equipped with the Video Measuring System DSA 1 and DSA-Software Version 1.5 For Windows '95 NT.

Dosing unit G1023 with G1060 1 ml syringe and G1061 needle (having a length of 30 mm and a diameter of 0.5 mm).

Microscope glass slides 10 mm x 30 mm.

### Conditioning oven

The system DSA 10 and the accessories DSA 1 with DSA-Software, G1023, G1060, G1061 are sold by Krüss GmbH, D 22453 Hamburg.

Preparation of the samples and contact angle measurements as_described below are conducted in the climatically controlled laboratory.

### Sample preparation

The preparation of the samples of the resins for the measurement of the equilibrium contact angle with water comprises the following steps:
1. prepare a 10% by weight solution of the resin in a suitable organic solvent. The high dilution provides a solution with a very low viscosity. A suitable solvent must be selected which dissolves the tackifying resins to be used in the context of the present invention, and which also promptly evaporates. A suitable solvent must therefore have a boiling point at atmospheric pressure not greater than 100°C, preferably not greater than 80°C. Tetrahydrofuran has been selected as a particularly suitable solvent according to the present test method;
2. completely dip a clean microscope glass slide (previously thoroughly cleaned with methanol and dried) into the solution;
3. remove the glass slide slowly from the solution and keep it vertical for about 10 seconds to obtain a uniform coating on both surfaces; the solvent almost immediately evaporates and leaves onto the glass surfaces a very thin, continuous layer of the resin having a uniform thickness and a smooth surface; samples which do not possess these features at a visual examination must be discarded;
4. let the sample dry at air for about ten minutes without touching the surfaces;
5. place the sample in the conditioning oven for at least 12 h at a temperature of 40°C and 85% relative humidity;
6. remove the sample from the conditioning oven and leave it cool in the climatic laboratory for about 20 minutes, until it reaches the room temperature of 25°C;
7. measure the equilibrium contact angle; it is not relevant on which side of the glass slide the contact angle measurement is actually performed, provided onto the selected side of the slide the resin coating is uniform and with a smooth surface, as explained under step (3).

### Contact angle measurement

Contact angle measurements are performed with the Drop Shape Analysis System DSA 10 equipped with the Video Measuring System DSA 1 with the DSA-Software, and the Dosing Unit G1023 with G1060 syringe and G1061 needle.

Liquid is distilled water used for HPLC (High Performance Liquid Chromatography).

Measurements of equilibrium contact angle are conducted according to the standard usage instructions provided with the DSA 10 system comprising the respective accessories listed above.

The measurements are performed at air and at room conditions, i.e. at 25°C and 55% relative humidity. The samples are positioned following the usage instructions onto the sample stage of the DSA 10 system.

The surface of the sample to be measured shall be horizontal.

A drop of water is positioned on the surface of the sample by means of the syringe G1060 with the G1061 needle. The drop volume has to be duly controlled by means of the micrometer dosing screw of the syringe such that the drop diameter ranges between 1÷2 mm. Within this range the contact angle is independent of the drop size.

The measurements of the equilibrium contact angle are conducted automatically by the Drop Shape Analysis System DSA 10 with the Video Measuring System DSA 1 and the DSA-Software. The Video Measuring System records a movie sequence of the drop positioned onto the sample surface, and the measurement is actually performed on the first frame in which the drop stands still on the surface of the sample in order to have the equilibrium contact angle.

For each sample, from five to seven different measurements are performed on different points of the surface. The highest and the lowest values obtained are discarded, and the remaining values are averaged, to ensure a representative equilibrium contact angle value to be determined for each sample under investigation.

The very thin layer of resin formed onto the glass slide according to the described sample preparation procedure, provides for an effective and reliable measurement of the equilibrium contact angle with water not only for resins which are solid at room temperature, but also for resins which are liquid at room temperature, wherein "solid" and "liquid" at room temperature are to be intended according to the definition previously given in the description. In the latter case in fact the glass slide provides the very thin resin layer of the sample with an effective support, and avoids any detrimental effect which might be caused by the higher density of the water_with respect to the resin. In other words, the combined effect of the very thin resin layer supported by the glass substrate, and of the very short time (few seconds) needed for the automatic measuring of the equilibrium contact angle after the drop has been positioned onto the sample surface, prevents any possible deformation of the horizontal and flat resin surface itself under the weight of the drop of water, and therefore provides for a correct equilibrium contact angle measurement.

## Claims

1. A disposable, moisture vapour permeable, liquid impermeable, covering sheet for bedding articles comprising at least a moisture vapour permeable fibrous layer and a liquid impermeable but moisture vapour permeable film layer of thermoplastic composition applied onto said fibrous layer, **characterised in that** said covering sheet has:
- a Water Vapour Transmission Rate (WVTR) value of at least 400 g/m²•24h;
- a Hydrostatic water Head (HH) value of at least 1 cm; and
- a ratio R of the caliper, expressed in micrometers to the basis weight, expressed in g/m², of at least 2.5,
said Water Vapour Transmission Rate, said Hydrostatic water Head, and said caliper measured according to the test methods described herein.

2. A disposable, moisture vapour permeable, liquid impermeable, covering sheet for bedding articles according to the claim 1 **characterised in that** said thermoplastic composition comprises:
a thermoplastic hydrophilic polymer or a mixture of thermoplastic hydrophilic polymers, said thermoplastic hydrophilic polymer, or alternatively each of said thermoplastic hydrophilic polymers of said mixture selected from the group consisting of polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, poly lactide and copolymers, polyamides, polyesters and copolyesters, polyester block copolymers, sulfonated polyesters,_poly-ether-ester block copolymers, poly-ether-ester-amide block copolymers, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, or mixtures thereof, and either
a compatible hydrophilic tackifying resin having an equilibrium contact angle with water not greater than 86 degrees, preferably not greater than 84 degrees, more preferably not greater than 82 degrees, as measured according to the Equilibrium Contact Angle Measuring Test described herein, or alternatively
a blend of compatible hydrophilic tackifying resins, said blend of compatible hydrophilic tackifying resins comprising at least 50% by weight of a tackifying resin or resins having an equilibrium contact angle with water not greater than 86 degrees, preferably not greater than 84 degrees, more preferably not greater than 82 degrees, as measured according to the Equilibrium Contact Angle Measuring Test described herein.

3. A disposable, moisture vapour permeable, liquid impermeable covering sheet for bedding articles according to the claim 2 wherein said thermoplastic composition further comprising a suitable compatible plasticiser, or a blend of suitable compatible plasticisers, wherein said suitable compatible plasticiser, or alternatively each plasticiser of said blend of suitable compatible plasticisers is selected from the group consisting of citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

4. A disposable, moisture vapour permeable, liquid impermeable, covering sheet for bedding articles according to any preceding claim, wherein said Water Vapour Transmission Rate (WVTR) is at least 500 g/m²•24h, preferably at least 600 g/m²•24h.

5. A disposable, moisture vapour permeable, liquid impermeable covering sheet for bedding articles according to any preceding claim, wherein said Hydrostatic water Head (HH) value is at least 10 cm, and/or said ratio R is at least 3.5.

6. A disposable, moisture vapour permeable, liquid impermeable, covering sheet for bedding articles according to any preceding claim wherein said covering sheet is a disposable mattress cover or a disposable pillow cover having a rectangular shape and a surface area from 15.0 m² to 0.3 m², preferably from 8.0 m² to 0.5 m².

7. A disposable mattress cover according to claim 6 having a surface area from 4.0 m² to 1.0 m².

8. A disposable mattress cover according to claim 7 having at least four adhesive means in form of stripes 3 .
